# EUROPEAN PATENT APPLICATION

(11) **EP 3 932 988 A1**
(43) Date of publication of application: **05.01.2022**
(21) Application number: 19918301.3
(22) Date of filing: 17.05.2019
(51) Int. Cl.: C08L 33/02, C08K 5/053, C08K 5/09, C08K 5/16

(54) **VISCOUS COMPOSITION**

(30) Priority: 01.03.2019 JP 2019037989
(71) Applicant: Sumitomo Seika Chemicals Co., Ltd., Kako-gun, Hyogo 675-0145 (JP)
(72) Inventor: YAMAGUCHI, Hirofumi, Himeji-shi, Hyogo 672-8076 (JP); UEZUMI, Chiaki, Himeji-shi, Hyogo 672-8076 (JP)
(74) Representative: Vossius & Partner Patentanwälte Rechtsanwälte mbB
(86) International application number: PCT/JP2019/019795
(87) International publication number: WO 2020/179092

(57) **Abstract**

Provided is a viscous composition that comprises a carboxyl-group-containing water-soluble copolymer, the composition having excellent viscosity even when an amino acid-based surfactant is contained in an amount greater than that of betaine. More specifically, provided is a viscous composition comprising (A) a copolymer obtained by polymerizing at least 100 parts by mass of a (meth)acrylic acid and 0.5 to 5 parts by mass of a (meth)acrylic acid alkyl ester having an alkyl group with 10 to 30 carbon atoms, (B) an amino acid-based surfactant, (C) a betaine, (D) an alkyl glycoside, and (E) an organic acid salt, wherein the mass content of (B) is equal to or greater than the mass content of (C).

## Description

### Technical Field

The present disclosure relates to a viscous composition and the like.

### Background Art

Water-soluble copolymers containing carboxyl groups are used in various fields as a thickening agent for cosmetics etc., a moisturizer for poultices etc., a suspension stabilizer for emulsions, suspensions, etc., and a gelling base for batteries etc.

In particular, for use as a composition for hair, a viscous composition that comprises a carboxyl-group-containing water-soluble copolymer preferably further contains a cleansing component and an active ingredient component useful for, for example, hair and/or scalp. Typical examples of such components include amino acid-based surfactants and betaines.

### Citation List

### Patent Literature

PTL 1: WO 2011/130460
PTL 2: WO 2014/021434

### Summary of Invention

### Technical Problem

However, a viscous composition that comprises an amino acid-based surfactant as well as betaine in addition to a carboxyl-group-containing water-soluble copolymer, in particular, an alkyl-modified carboxyl-group-containing water-soluble copolymer, has a significantly decreased viscosity when the amino acid-based surfactant is contained in an amount equal to or greater than that of betaine. Therefore, producing a viscous composition that comprises a carboxyl-group-containing water-soluble copolymer and has excellent viscosity was difficult when the amino acid-based surfactant is contained in an amount equal to or greater than that of betaine. In Patent Literature (PTL) 1, a viscous composition that can achieve a high viscosity with the use of an alkyl-modified carboxyl-group-containing water-soluble copolymer in the acidic to weakly acidic pH region, even in the presence of a polyvalent metal salt, is analyzed. However, this composition does not contain an amino acid-based surfactant and betaine. In addition, in PTL 2, a viscous composition that comprises a specific carboxyl-group-containing water-soluble copolymer and a specific amino acid surfactant is analyzed. However, this composition does not contain the amino acid-based surfactant in an amount equal to or greater than that of betaine.

### Solution to Problem

The present inventors prepared a viscous composition by blending an alkyl-modified carboxyl-group-containing water-soluble copolymer, an amino acid-based surfactant, and betaine, and further blending an alkyl glycoside and an organic acid salt, and thereby found the possibility of preparing a viscous composition that comprises a carboxyl-group-containing water-soluble copolymer with excellent viscosity, even when the amino acid-based surfactant is contained in an amount equal to or greater than that of betaine. The present inventors then made further improvements.

The present disclosure encompasses, for example, the subject matter described in the following items.

### Item 1.

### A viscous composition comprising

(A) a copolymer obtained by polymerizing at least 100 parts by mass of a (meth)acrylic acid and 0.5 to 5 parts by mass of a (meth)acrylic acid alkyl ester having an alkyl group with 10 to 30 carbon atoms,
(B) an amino acid-based surfactant,
(C) a betaine,
(D) an alkyl glycoside, and
(E) an organic acid salt,
wherein the mass content of (B) is equal to or greater than the mass content of (C).

### Item 2.

The viscous composition according to Item 1, having a viscosity of 1000 mPa·s or more.

### Item 3.

The viscous composition according to Item 1 or 2, wherein (A) is a copolymer obtained by polymerizing at least 100 parts by mass of a (meth)acrylic acid and 0.5 to 5 parts by mass of a (meth)acrylic acid alkyl ester having an alkyl group with 18 to 24 carbon atoms.

### Item 4.

The viscous composition according to any one of Items 1 to 3, wherein (A) is a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and a compound with two or more ethylenically unsaturated groups.

### Item 5.

The viscous composition according to Item 4, wherein (A) is a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and 0.001 to 1 part by mass of a compound with two or more ethylenically unsaturated groups.

### Item 6.

The viscous composition according to Item 4 or 5, wherein the compound with two or more ethylenically unsaturated groups is at least one member selected from the group consisting of pentaerythritol allyl ether, tetraallyloxyethane, triallyl phosphate, and polyallylsaccharose.

### Item 7.

The viscous composition according to any one of Items 1 to 6, wherein the content mass ratio of (B) and (C) is 1 to 5:1.

### Item 8.

The viscous composition according to any one of Items 1 to 7, wherein (B) is a compound represented by the formula: 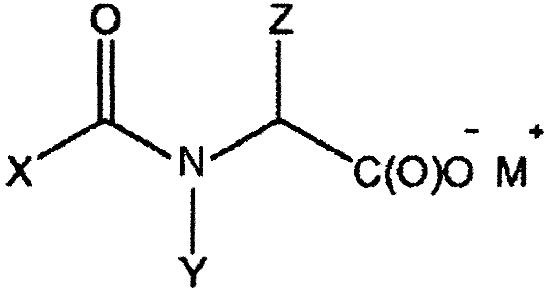
wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O⁻M⁺, -CH₂COOH, or -(CH₂)₂COOH, and
M represents sodium, potassium, ammonium, or triethanolammonium.

### Item 9.

The viscous composition according to any one of Items 1 to 8, wherein (C) is at least one member selected from the group consisting of compounds represented by the formula: 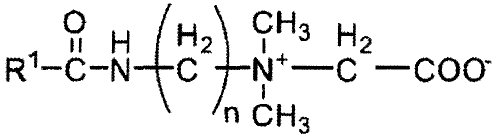 wherein R¹ represents a linear or branched alkyl group with 5 to 22 carbon atoms, and n represents 1 to 6, and
compounds represented by the formula: 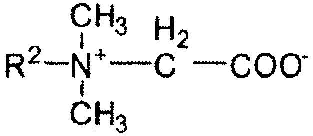 wherein R² represents a linear or branched alkyl group with 1 to 22 carbon atoms.

### Item 10.

The viscous composition according to any one of Items 1 to 7,
wherein (B) is a compound represented by the formula: 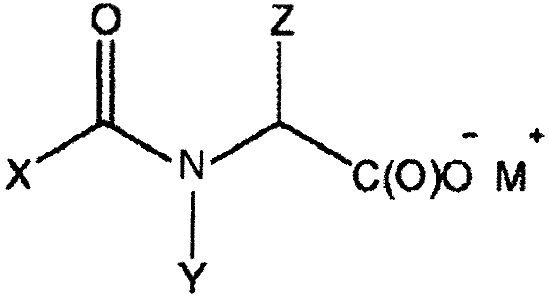
wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O⁻M⁺, -CH₂COOH, or - (CH₂) ₂COOH, and
M represents sodium, potassium, ammonium, or triethanolammonium; and
wherein (C) is at least one member selected from the group consisting of compounds represented by the formula: 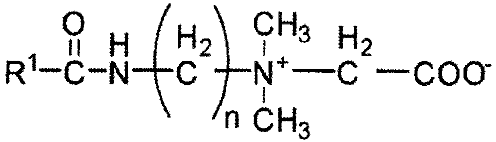 wherein R¹ represents a linear or branched alkyl group with 5 to 22 carbon atoms, and n represents 1 to 6, and compounds represented by the formula: 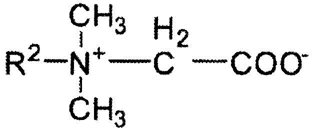
wherein R² represents a linear or branched alkyl group with 1 to 22 carbon atoms.

### Item 11.

The viscous composition according to any of Items 1 to 10, wherein (D) is an alkyl glucoside in which one linear alkyl group with 5 to 22 carbon atoms and one glucose are condensed.

### Item 12.

The viscous composition according to any of Items 1 to 11, wherein (E) is at least one member selected from the group consisting of carboxylic acid metal salts and ascorbic acid metal salts.

### Item 13.

The viscous composition according to Item 6, wherein
the content mass ratio of (B) and (C) is 1 to 5:1,
the compound with two or more ethylenically unsaturated groups is at least one member selected from the group consisting of pentaerythritol allyl ether, tetraallyloxyethane, triallyl phosphate, and polyallylsaccharose,
(B) is a compound represented by the formula:

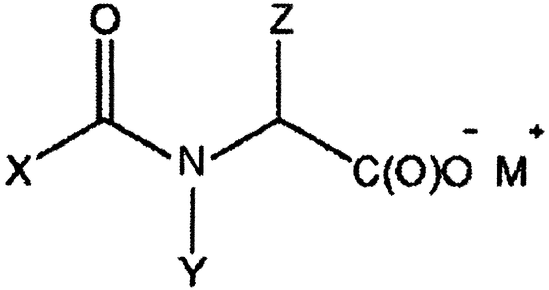

wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O⁻M⁺, -CH₂COOH, or - (CH₂) ₂COOH, and
M represents sodium, potassium, ammonium, or triethanolammonium,
(C) is at least one member selected from the group consisting of compounds represented by the formula: 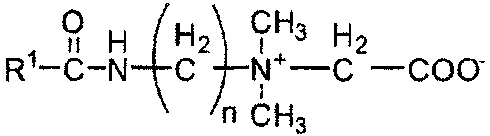
wherein R¹ represents a linear or branched alkyl group with 5 to 22 carbon atoms, and n represents 1 to 6, and compounds represented by the formula: 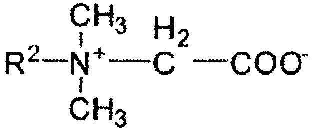
wherein R² represents a linear or branched alkyl group with 1 to 22 carbon atoms,
(D) is an alkyl glucoside in which one linear alkyl group with 5 to 22 carbon atoms and one glucose are condensed, and
(E) is at least one member selected from the group consisting of carboxylic acid metal salts and ascorbic acid metal salts.

### Advantageous Effects of Invention

Provided is a viscous composition that comprises a carboxyl-group-containing water-soluble copolymer, the composition having excellent viscosity even when an amino acid-based surfactant is contained in an amount greater than that of betaine.

### Description of Embodiments

Embodiments encompassed by the present disclosure are described in more detail below. The present disclosure preferably encompasses, for example, a viscous composition (in particular, a composition for hair) and a method for preparing a viscous composition. However, the present disclosure is not limited to these and encompasses everything disclosed in this specification and recognizable to those skilled in the art.

The viscous composition encompassed by the present disclosure comprises the following (A) to (E):
(A) a copolymer obtained by polymerizing at least 100 parts by mass of a (meth)acrylic acid and 0.5 to 5 parts by mass of a (meth)acrylic acid alkyl ester having an alkyl group with 10 to 30 carbon atoms,
(B) an amino acid-based surfactant,
(C) a betaine,
(D) an alkyl glycoside, and
(E) an organic acid salt.

In this specification, the viscous composition encompassed by the present disclosure may be referred to as "the viscous composition of the present disclosure." The viscous composition of the present disclosure can be prepared by using a liquid composition that comprises, for example, (A) to (E) and water, as described below. When prepared in this manner, the viscous composition of the present disclosure further comprises water (F).

As stated above, (A) is a copolymer obtained by polymerizing at least 100 parts by mass of a (meth)acrylic acid and 0.5 to 5 parts by mass of a (meth)acrylic acid alkyl ester having an alkyl group with 10 to 30 carbon atoms (an alkyl-modified carboxyl-group-containing water-soluble copolymer). (A) is preferably a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and a compound with two or more ethylenically unsaturated groups; and more preferably a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and 0.001 to 1 part by mass of a compound with two or more ethylenically unsaturated groups.

In this specification, the term "(meth)acrylic" means acrylic and/or methacrylic. For the (meth)acrylic acid, either acrylic acid or methacrylic acid may be used alone, or used in combination.

The (meth)acrylic acid alkyl ester having an alkyl group with 10 to 30 (10, 11, 12, 13, 14, 15, 16, 17, 18, 19, 20, 21, 22, 23, 24, 25, 26, 27, 28, 29, or 30) carbon atoms refers to an ester of (meth)acrylic acid and a higher alcohol having an alkyl group with 10 to 30 carbon atoms. The carbon number of this alkyl group is more preferably, for example, 12 to 30, 14 to 28, 16 to 26, or 18 to 24. Examples of the (meth)acrylic acid alkyl ester include, but are not particularly limited to, esters of (meth)acrylic acid with stearyl alcohol, esters of (meth)acrylic acid with eicosanol, esters of (meth)acrylic acid with behenyl alcohol, and esters of (meth)acrylic acid with tetracosanol. Among these (meth)acrylic acid alkyl esters, stearyl methacrylate, eicosanyl methacrylate, behenyl methacrylate, and tetracosanyl methacrylate are preferred. These (meth)acrylic acid alkyl esters may be used alone, or in a combination of two or more. These (meth)acrylic acid alkyl esters for use may be commercial products, such as BLEMMER VMA-70, product name, produced by NOF Corporation.

The amount of the (meth)acrylic acid alkyl ester for use is 0.5 to 5 parts by mass, preferably 0.5 to 4 parts by mass or 1 to 4 parts by mass, and more preferably 1 to 3 parts by mass, per 100 parts by mass of the (meth)acrylic acid. If the amount of the (meth)acrylic acid alkyl ester for use is less than 0.5 parts by mass, mamako (aggregate) tends to be easily generated when the resulting alkyl-modified carboxyl-group-containing water-soluble copolymer is dispersed in water. If the amount of the (meth)acrylic acid alkyl ester for use exceeds 5 parts by mass, the solubility of the resulting alkyl-modified carboxyl-group-containing water-soluble copolymer tends to be poor when used to prepare a viscous composition.

Examples of the compound with two or more ethylenically unsaturated groups include but are not particularly limited to, a compound in which a polyol, such as ethylene glycol, propylene glycol, polyoxyethylene glycol, polyoxypropylene glycol, glycerol, polyglycerol, trimethylolpropane, pentaerythritol, saccharose, or sorbitol is substituted with two or more acrylic acid esters; a compound in which a polyol mentioned above is substituted with two or more allyl ethers; and diallyl phthalate, triallyl phosphate, allyl methacrylate, tetraallyloxyethane, triallyl cyanurate, divinyl adipate, vinyl crotonate, 1,5-hexadiene, divinyl benzene, and polyallylsaccharose. Among these, the compound with two or more ethylenically unsaturated groups is preferably pentaerythritol allyl ether (more preferably pentaerythritol triallyl ether or pentaerythritol tetraallyl ether), tetraallyloxyethane, triallyl phosphate, or polyallylsaccharose, since the use of a small amount of the resulting alkyl-modified carboxyl-group-containing water-soluble copolymer can produce a viscous composition with high thickening properties, and since high suspension stability can be imparted to emulsions, suspensions, etc. These compounds with two or more ethylenically unsaturated groups may be used alone, or in a combination of two or more.

In preparing the copolymer of (A), the amount of the compound with two or more ethylenically unsaturated groups, when also used in the polymerization, is not particularly limited and is preferably 0.001 to 1 part by mass per 100 parts by mass of the (meth)acrylic acid. The lower limit of this range may be, for example, 0.005, 0.01, 0.015, 0.02, 0.025, 0.03, 0.035, or 0.04. The upper limit of this range may be, for example, 0.95, 0.9, 0.85, 0.8, 0.75, 0.7, 0.65, or 0.6. For example, the amount for use is preferably 0.01 to 0.8 parts by mass, and more preferably 0.02 to 0.7 parts by mass, per 100 parts by mass of the (meth)acrylic acid.

In the production of the copolymer of (A), the method of polymerizing (meth)acrylic acid and the (meth)acrylic acid alkyl ester, and optionally the compound with two or more ethylenically unsaturated groups is not particularly limited. Examples include a method of polymerizing these in a polymerization solvent in the presence of a radical polymerization initiator.

Examples of the radical polymerization initiator include, but are not particularly limited to, α,α'-azobisisobutyronitrile, 2,2'-azobis-2,4-dimethylvaleronitrile, 2,2'-azobis methyl isobutyrate, benzoyl peroxide, lauroyl peroxide, cumene hydroperoxide, and tertiary butyl hydroperoxide. These radical polymerization initiators may be used alone, or in a combination of two or more.

The amount of the radical polymerization initiator for use is not particularly limited and is preferably, for example, 0.01 to 0.45 parts by mass, and more preferably 0.01 to 0.35 parts by mass, per 100 parts by mass of the (meth)acrylic acid. When the radical polymerization initiator is used in this range, the polymerization reaction speed can be appropriately controlled, making it possible to economically produce an alkyl-modified carboxyl-group-containing water-soluble copolymer.

For the polymerization, components other than those listed above (sometimes referred to as "components added during polymerization") may further be used. Examples of the components added during polymerization include surfactants (except for those of (A) to (E) components). These surfactants are preferably, for example, known surfactants that are known to be used for emulsion polymerization. Preferable examples of such surfactants include nonionic surfactants with one or more polyoxyethylene chains.

Preferable examples of the nonionic surfactant with one or more polyoxyethylene chains include polyhydric alcohol fatty acid ester-ethylene oxide adducts, block copolymers of hydroxy fatty acids and ethylene oxide, and polyoxyethylene castor oil.

Preferable examples of the polyhydric alcohol fatty acid ester-ethylene oxide adducts include ester compounds of polyoxyethylene hydrogenated castor oil and polyhydric alcohol fatty acids. The polyhydric alcohol fatty acid here is preferably a saturated or unsaturated polyhydric (in particular, divalent) alcohol fatty acid with 14 to 24 (14, 15, 16, 17, 18, 19, 20, 21, 22, 23, or 24) carbon atoms. More specifically, for example, isopalmitic acid, isostearic acid, and isooleic acid are preferred. The average number of moles of ethylene oxide added to constitute polyoxyethylene in ester compounds of polyoxyethylene hydrogenated castor oil and polyhydric alcohol fatty acids is not particularly limited, and may be, for example, about 20 to 100 or about 30 to 70. Particularly preferable examples of the ester compounds of polyoxyethylene hydrogenated castor oil and polyhydric alcohol fatty acids include polyoxyethylene hydrogenated castor oil isostearates.

In polyoxyethylene castor oil, the number of moles of ethylene oxide added is preferably about 2 to 10, and more preferably about 2 to 5.

The block copolymer of a hydroxy fatty acid and ethylene oxide can be rephrased as a copolymer of a polyhydroxy fatty acid and polyoxyethylene. The fatty acid of polyhydroxy fatty acid is preferably a fatty acid with 14 to 22 carbon atoms. Preferable examples include myristic acid, palmitic acid, and stearic acid. The hydroxy fatty acid is preferably, for example, hydroxymyristic acid, hydroxypalmitic acid, or hydroxystearic acid, and particularly preferably hydroxystearic acid. The hydroxystearic acid is particularly preferably 12-hydroxystearic acid. The polyhydroxy fatty acid is particularly preferably a polyhydroxystearic acid. The block copolymer of a hydroxy fatty acid and ethylene oxide is particularly preferably a block copolymer of 12-hydroxystearic acid and ethylene oxide.

The surfactants may be used alone, or in a combination of two or more.

The polymerization solvent is not particularly limited, and is preferably a solvent that dissolves (meth)acrylic acid, the (meth)acrylic acid alkyl ester, and the compound with two or more ethylenically unsaturated groups, and that does not dissolve the resulting alkyl-modified carboxyl-group-containing water-soluble copolymer. Specific examples of such polymerization solvents include normal pentane, normal hexane, normal heptane, normal octane, isooctane, cyclopentane, methylcyclopentane, cyclohexane, methylcyclohexane, benzene, toluene, xylene, chlorobenzene, ethylene dichloride, ethyl acetate, isopropyl acetate, ethyl methyl ketone, and isobutyl methyl ketone. Among these polymerization solvents, ethylene dichloride, normal hexane, normal heptane, and ethyl acetate are preferred from the standpoint of stable quality and easy availability. These polymerization solvents may be used alone, or in a combination of two or more.

The amount of the polymerization solvent for use is not particularly limited, and is preferably, for example, 200 to 10,000 parts by mass, and more preferably 300 to 2,000 parts by mass, per 100 parts by mass of the (meth) acrylic acid. The use of the polymerization solvent within this range suppresses aggregation of the alkyl-modified carboxyl-group-containing water-soluble copolymer even when the polymerization reaction proceeds, allowing stirring to be performed uniformly, and the polymerization reaction to proceed efficiently.

The atmosphere during the polymerization reaction is not particularly limited as long as the polymerization reaction can proceed. Examples include an inert gas atmosphere, such as nitrogen gas or argon gas.

The reaction temperature during the polymerization reaction is not particularly limited as long as the polymerization reaction can proceed, and is preferably, for example, 50 to 90°C, and more preferably 55 to 75°C. When the polymerization reaction is performed within this reaction temperature range, an increase in the reaction solution viscosity can be suppressed, and the reaction can be easily controlled; additionally, the bulk density of the resulting alkyl-modified carboxyl-group-containing water-soluble copolymer can be controlled.

The reaction time for the polymerization reaction cannot be generalized because it varies depends on the reaction temperature. The reaction time is typically 2 to 10 hours.

After completion of the reaction, for example, the reaction solution is heated to 80 to 130°C, and the polymerization solvent is removed, whereby a white fine powder of an alkyl-modified carboxyl-group-containing water-soluble copolymer can be isolated.

In the viscous composition of the present disclosure, the alkyl-modified carboxyl-group-containing water-soluble copolymers may be used alone, or in a combination of two or more.

The amino acid-based surfactant (B) is preferably one in which the amine group located at the α-carbon of an amino acid salt is a carboxylic acid salt of α-amino acid acylated with a C6-C22 fatty acid derivative. Such amino acid-based surfactants are particularly useful for a mild, high-foaming cleaning agent. More specific examples include surfactants represented by the following formula: 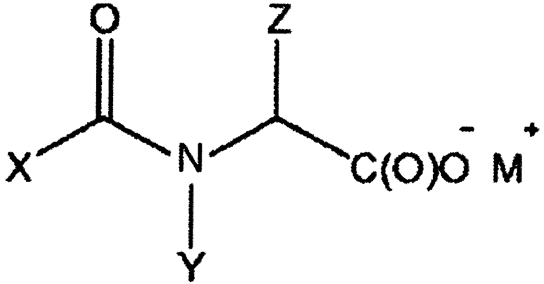
wherein X represents a saturated or unsaturated hydrocarbon group having 5 to 22 (5, 6, 7, 8, 9, 10, 11, 12, 13, 14, 15, 16, 17, 18, 19, 20, 21, or 22) carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O⁻M⁺, -CH₂COOH, or - (CH₂)₂COOH, and
M⁺ represents a salt-forming cation.

In one embodiment, X represents a group selected from linear or branched C5-C22 alkyl groups and linear or branched C5-C22 alkenyl groups. In one embodiment, M is selected from sodium, potassium, ammonium, and triethanolammonium.

Specific examples of the amino acid-based surfactant (B) include mono- and dicarboxylic acid salts (e.g., sodium, potassium, ammonium, and triethanolammonium) of N-acylated glutamic acid, such as sodium cocoyl glutamate, sodium lauroyl glutamate, sodium myristoyl glutamate, sodium palmitoyl glutamate, sodium stearoyl glutamate, disodium cocoyl glutamate, disodium stearoyl glutamate, potassium cocoyl glutamate, potassium lauroyl glutamate, and potassium myristoyl glutamate; carboxylic acid salts (e.g., sodium, potassium, ammonium, and triethanolammonium) of N-acylated alanine, such as sodium cocoyl alaninate, TEA-cocoyl alaninate, sodium cocoyl methylalanine, sodium lauroyl alanine, and TEA lauroyl alaninate; carboxylic acid salts (e.g., sodium, potassium, ammonium, and triethanolammonium) of N-acylated glycine, such as sodium cocoyl glycinate and potassium cocoyl glycinate; carboxylic acid salts (e.g., sodium, potassium, ammonium, and triethanolammonium) of N-acylated sarcosine, such as sodium lauroyl sarcosinate, sodium cocoyl sarcosinate, sodium myristoyl sarcosinate, sodium oleoyl sarcosinate, and ammonium lauroyl sarcosinate. Among these, sodium cocoyl glutamate, potassium cocoyl glutamate, sodium cocoyl alaninate, potassium cocoyl alaninate, sodium cocoyl glycinate, and potassium cocoyl glycinate are more preferred.

The amino acid-based surfactant (B) can be used alone, or in a combination of two or more.

The betaine (C) is preferably, for example, amido betaine, alkyl betaine, alkylamidopropyl betaine, cocobetaine, or alkylsulfobetaine (sultaine). The alkyl and acyl group here have 5 to 22 (5, 6, 7, 8, 9, 10, 11, 12, 13, 14, 15, 16, 17, 18, 19, 20, 21, or 22), and preferably 7 to 19 carbon atoms.

More specific examples of amido betaine include betaines represented by the following formula: 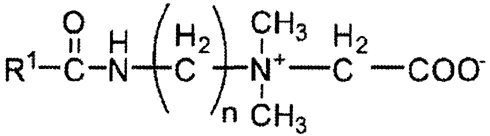 wherein R¹ represents a linear or branched alkyl group with 5 to 22 (5, 6, 7, 8, 9, 10, 11, 12, 13, 14, 15, 16, 17, 18, 19, 20, 21, or 22) carbon atoms. The betaine may also be a mixture of betaines with different R¹, and preferably include, for example, a betaine in which R¹-CO- represents a coconut oil fatty acid residue. Also, n represents 1 to 6 (1, 2, 3, 4, 5, or 6), and particularly preferably 3.

More specific examples of the betaine include coconut oil fatty acid amidopropyl betaine (i.e., cocamidopropyl betaine: CAPB) and lauric acid amidopropyl betaine.

More specific examples of alkyl betaine include betaines represented by the following formula: 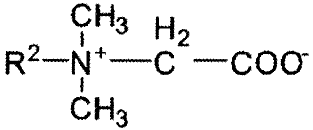 wherein R² represents a linear or branched alkyl group with 1 to 22 (1, 2, 3, 4, 5, 6, 7, 8, 9, 10, 11, 12, 13, 14, 15, 16, 17, 18, 19, 20, 21, or 22) carbon atoms.

More specific examples of the betaine include trimethylaminoacetic acid betaine (trimethylglycine), lauryldimethylacetic acid betaine, and tetradecyl dimethylamino acetic acid betaine.

The betaine (C) may be used alone, or in a combination of two or more.

The alkyl glycoside (D) is preferably a condensation product of a sugar with a linear or branched alkyl group with 5 to 22 (5, 6, 7, 8, 9, 10, 11, 12, 13, 14, 15, 16, 17, 18, 19, 20, 21, or 22) carbon atoms. The alkyl chain here more preferably has 8 to 22 or 10 to 20 carbon atoms, and still more preferably 12 to 20 carbon atoms. The alkyl group is more preferably linear. The sugar is preferably, for example, glucose. The condensation product may be one in which one or more of such alkyl groups and a sugar are condensed, and is preferably one in which one alkyl group and one sugar are condensed.

More specific examples of the alkyl glycoside include an alkyl glucoside in which one linear alkyl group with 8 to 22 carbon atoms and one glucose are condensed (linked by glycosidic bond). Furthermore specific examples include n-octyl-β-D-glucoside, n-octyl-β-D-maltoside, n-decyl-β-D-glucoside (decyl glucoside), n-decyl-β-D-maltoside, and n-dodecyl-β-D-glucoside (lauryl glucoside).

The alkyl glycoside (D) may be used alone, or in a combination of two or more.

Preferable examples of the organic acid salt (E) include carboxylic acid salts, amino acid salts, and ascorbic acid salts. Among these, carboxylic acid salts are preferred. The carboxylic acid of the carboxylic acid salts may be monovalent or multivalent (e.g., divalent, trivalent, or tetravalent). Specifically, for example, monocarboxylic acid, dicarboxylic acid, tricarboxylic acid, and tetracarboxylic acid are preferred. More specific examples include acetic acid, butyric acid, lactic acid, benzoic acid, gluconic acid, oxalic acid, malonic acid, succinic acid, glutaric acid, adipic acid, phytic acid, fumaric acid, maleic acid, tartaric acid, malic acid, phthalic acid, citric acid, and ethylenediaminetetraacetic acid.

The organic acid salt (E) is preferably a metal salt. Examples of the metal salt include those that form a monovalent or multivalent (e.g., divalent, trivalent, or tetravalent) metal ions in an aqueous solution. Specific examples include sodium salts, potassium salts, magnesium salts, calcium salts, strontium salts, barium salts, radium salts, zinc salts, and aluminum salts.

Specifically, among the above, the organic acid salt (E) is preferably, but not particularly limited to, for example, sodium acetate and sodium citrate (in particular, trisodium citrate).

The organic acid salt (E) may be used alone, or in a combination of two or more.

The viscous composition of the present disclosure may comprise an inorganic acid salt as long as the effects of the viscous composition of the present disclosure are not impaired; however, the viscous composition of the present disclosure preferably does not comprise an inorganic acid salt.

In the viscous composition of the present disclosure, (A) is preferably contained in an amount of, for example, about 0.1 to 5 mass%. The lower limit of the range may be, for example, 0.2, 0.3, 0.4, 0.5, 0.6, 0.7, 0.8, or 0.9 mass%. The upper limit of the range may be, for example, 4.5, 4, 3.5, 3, 2.5, 2, or 1.5. For example, (A) is more preferably contained in an amount of 0.2 to 4 mass%, and still more preferably 0.5 to 2 mass%.

In the viscous composition of the present disclosure, (B) is preferably contained in an amount of, for example, 1 to 10 parts by mass, more preferably 2 to 8 parts by mass, still more preferably 3 to 7 parts by mass, and even more preferably 4 to 6 parts by mass, per part by mass of (A). Further, (B) is preferably contained in the viscous composition of the present disclosure in an amount of, for example, 1 to 10 mass%, more preferably 2 to 10 mass%, and still more preferably 3 to 10 mass%. The upper limit of this range may be, for example, 9, 8, or 7 mass%.

In the viscous composition of the present disclosure, (C) is preferably contained in an amount of, for example, 0.5 to 10 parts by mass, more preferably 1 to 8 parts by mass, and still more preferably 1.5 to 7 parts by mass, per part by mass of (A). The lower limit of this range may be 2 or 3, and the upper limit of this range may be 6. Further, (C) is preferably contained in the viscous composition of the present disclosure in an amount of, for example, 0.5 to 10 mass%, more preferably 1 to 8 mass%, and still more preferably 1.5 to 7 mass%.

Accordingly, in the viscous composition of the present disclosure, the mass content of (B) is equal to or greater than the mass content of (C). Although there are no particular limitations, for example, the content mass ratio of (B) and (C) is preferably 1 to 5:1, and more preferably 1 to 4:1 or 1 to 3:1.

In the viscous composition of the present disclosure, (D) is preferably contained in an amount of, for example, 0.5 to 10 parts by mass, more preferably 1 to 8 parts by mass, and still more preferably 1 to 7 parts by mass, per part by mass of (A). The lower limit of this range may be 2 or 3, and the upper limit of this range may be 6. Further, (D) is preferably contained in the viscous composition of the present disclosure in an amount of, for example, 0.5 to 10 mass%, more preferably 1 to 8 mass%, and still more preferably 1 to 7 mass%.

In the viscous composition of the present disclosure, (E) is preferably contained in an amount of, for example, 1 to 10 parts by mass, more preferably 2 to 8 parts by mass, still more preferably 3 to 7 parts by mass, and even more preferably 3 to 6 parts by mass, per part by mass of (A). Further, (B) is contained in the viscous composition of the present disclosure in an amount of, for example, 1 to 10 mass%, more preferably 2 to 8 mass%, still more preferably 3 to 7 mass%, and even more preferably 3 to 6 mass%.

The viscous composition of the present disclosure has a viscosity of preferably 1000 mPa·s or more, and more preferably 1000 to 50000 mPa·s. The upper limit of the range may be, for example, 45000, 40000, 35000, 30000, 25000, 20000, 15000, or 10000 mPa·s. The upper limit of the range may also be 9000, 8000, 7000, 6000, 5000, or 4000 mPa·s. For example, the viscosity is more preferably about 1000 to 20000 mPa·s, and still more preferably about 1000 to 10000 mPa·s. The viscosity is measured at an ordinary temperature (25°C) using a Brookfield viscometer (model number: DV1MRVTJO) at a rotation speed of 20 revolutions per minute. For rotors used in the measurement, rotor No. 3 is used for the viscosity of less than 2000 mPa·s, rotor No. 4 is used for the viscosity of 2000 mPa·s or more and less than 5000 mPa·s, rotor No. 5 is used for the viscosity of 5000 mPa·s or more and less than 15000 mPa-s, rotor No. 6 is used for the viscosity of 15000 mPa·s or more and less than 40000 mPa·s, and rotor No. 7 is used for the viscosity of 40000 mPa·s or more.

The viscous composition of the present disclosure is preferably highly transparent. Specifically, the viscous composition of the present disclosure has a transmittance of light with a wavelength of 425 nm of 20% or more, more preferably 25 or 30% or more, and still more preferably 35 or 40% or more. The transmittance refers to a value obtained by defoaming the viscous composition and then performing measurement at a measurement wavelength of 425 nm using a cell with an optical path length of 1 cm (i.e., the percentage of an amount of transmission at 425 nm, taking that of pure water as 100%).

The viscous composition of the present disclosure preferably has a pH of 4 to 6.5, more preferably 4.5 to 6, and even more preferably 5 to 6. The pH value is measured using a pH meter at an ordinary temperature (25°C).

The viscous composition of the present disclosure can be prepared, for example, by preparing in advance an aqueous dispersion of (A) dispersed in water, an aqueous solution of (B), an aqueous solution of (C), an aqueous solution of (D), and an aqueous solution of (E), and mixing these components in sequence by stirring. The order of mixing is not particularly limited. The mixing may be performed sequentially, for example, from (A) to (B), then to (C), then to (D), and then to (E). The order of mixing of (A) to (D) can be set as appropriate. It is preferable that (E) is mixed last.

In addition to the above components, the viscous composition of the present disclosure may further comprise other additives and active ingredients according to its application as long as the effects of the present disclosure are not impaired. For example, when used as cosmetics, the viscous composition of the present disclosure may comprise a moisturizer, an antioxidant, a blood circulation stimulant, a cooling agent, an antiperspirant, a disinfectant, a skin activator, a deodorant, a surfactant, a fragrance, a dye, and the like. In particular, from the standpoint of eliminating stickiness to the skin and further improving the excellent feeling of smoothness, the viscous composition of the present disclosure, when used as cosmetics, preferably comprises, for example, a moisturizer, such as glycerol, propylene glycol, dipropylene glycol, 1,3-butylene glycol, polyethylene glycol, sorbitol, sodium lactate, sodium 2-pyrrolidone-5-carboxylate, sodium hyaluronate, and sodium acetylated hyaluronate.

When other additives or active ingredients are used to produce the viscous composition of the present disclosure, these additives or active ingredients may be added, for example, during the sequential mixing of (A) to (E) described above.

The viscous composition of the present disclosure has the viscosity mentioned above, and is in the form of, for example, a viscous liquid, flowable gel, or cream.

The viscous composition of the present disclosure is used in the fields of, for example, cosmetics, pharmaceuticals (in particular, preparation for external use for skin), toiletry products, household products, and water-soluble paint. In particular, the viscous composition of the present disclosure, which has high viscosity, and which can impart a smooth and fresh feeling while suppressing stickiness when applied to the skin, is suitable for use in cosmetics, preparations for external use for skin, or toiletry products.

When the viscous composition of the present disclosure is used for cosmetics, the form of the formulation is not particularly limited. Examples include lotions, emulsions, serums, creams, cream pack, massage creams, hair-setting gels, sunscreens, styling gels, eyeliners, mascaras, lipsticks, and foundations. When the viscous composition of the present disclosure is used as a toiletry product, the form of the formulation is not particularly limited. Examples include cleansing creams, cleansing gels, facial cleansing foams, hair washes, body washes, and hair conditioners.

In this specification, the terms "comprising" and "containing" include "consisting essentially of" and "consisting of." Further, the present disclosure includes any combination of the constituent requirements described in this specification.

In addition, the various characteristics (properties, structures, functions, etc.) described in each embodiment of the present disclosure described above may be combined in any way in specifying the subjects included in the present disclosure. In other words, the present disclosure includes all the subjects comprising all combinations of the combinable characteristics described in this specification.

### Examples

The present disclosure is described in more detail below. However, the present disclosure is not limited to the following Examples.

### Measurement Methods

The viscosity, pH, and transmittance of the viscous compositions obtained in Examples and Comparative Examples below were evaluated according to the following methods.

### (1) Viscosity

After immersing an evaluation sample (each viscous composition, the same applies below) for 60 minutes or more in a constant-temperature water tank adjusted to 25°C, the viscosity was measured using a Brookfield viscometer (model number: DV1MRVTJ0) at 25°C after 1-minute rotation at a rotation speed of 20 revolutions per minute. For the measurement, rotor No. 3 was used for the viscosity of less than 2000 mPa·s, rotor No. 4 was used for the viscosity of 2000 mPa·s or more and less than 5000 mPa·s, rotor No. 5 was used for the viscosity of 5000 mPa·s or more and less than 15000 mPa·s, rotor No. 6 was used for the viscosity of 15000 mPa·s or more and less than 40000 mPa·s, and rotor No. 7 was used for the viscosity of 40000 mPa·s or more. A sample with a viscosity of 1000 mPa·s or more is capable of exhibiting the characteristic of not easily dripping during application, and thus can be evaluated to be an excellent, high-viscosity sample.

### (2) pH

The pH of the evaluation samples was measured at room temperature (25°C) using a pH meter (model number: D-51) produced by HORIBA, Ltd. Specifically, an electrode of the pH meter was inserted into the sample, and the MEAS key was pressed. When the HOLD display changed from flashing to lit, the displayed value was read and recorded.

### (3) Transmittance

The transmittance (%) of the evaluation sample was measured using a spectrophotometer (model number: UV-1850) produced by Shimadzu Corp. First, the sample was placed in a cell for UV measurement (optical path length: 1 cm) and defoamed in a centrifuge at 2,000 revolutions per minute for 5 minutes. If defoaming was not completed, the same operation was performed in the centrifuge to confirm that the foam was completely removed from the upper part of the sample. The sample was then placed in a spectrophotometer, and the transmittance was measured at a measurement wavelength of 425 nm. For the use of a viscous composition, the degree of transmittance is usually not a great concern. However, when used, in particular, for cosmetics, transparency and no cloudiness can be required. Thus, a higher transmittance is considered to be more preferable.

### Production Example 1

45 g (0.625 mol) of acrylic acid, 1.35 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), 0.02 g of pentaerythritol tetraallyl ether, 150 g of normal hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the solvent. Then, the mixture was reacted for 4 hours in a nitrogen atmosphere while the temperature was maintained at 60 to 65°C. After completion of the reaction, the resulting slurry was heated to 90°C to distill off the normal hexane, and further dried under reduced pressure for 8 hours (110°C, 10 mmHg) to thus obtain 43 g of a white fine powder of an alkyl-modified carboxyl-group-containing water-soluble copolymer. This copolymer is sometimes referred to below as "the polymer of Production Example 1."

### Production Example 2

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.19 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 39 g of a white fine powder of an alkyl-modified carboxyl-group-containing water-soluble copolymer. This copolymer is sometimes referred to below as "the polymer of Production Example 2."

### Production Example 3

40 g of acrylic acid, 0.88 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 0.26 g of polyoxyethylene castor oil (produced by Nikko Chemicals Co., Ltd., product name: CO-3, an adduct in which 3 mol of ethylene oxide is added), and 0.98 g of polyoxyethylene hydrogenated castor oil triisostearate (Nihon Emulsion Co., Ltd., product name: RWIS-350, an adduct in which 50 mol of ethylene oxide is added) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 38 g of a white fine powder of an alkyl-modified carboxyl-group-containing water-soluble copolymer. This copolymer is sometimes referred to below as "the polymer of Production Example 3."

### Production Example 4

40 g of acrylic acid, 0.4 g of BLEMMER VMA-70 (produced by NOF Corporation, a mixture of 10 to 20 parts by mass of stearyl methacrylate, 10 to 20 parts by mass of eicosanyl methacrylate, 59 to 80 parts by mass of behenyl methacrylate, and 1 mass% or less of tetracosanyl methacrylate), 0.22 g of pentaerythritol tetraallyl ether, 0.116 g of 2,2'-azobis(methyl isobutyrate), and 230.9 g of normal hexane were placed in a 500-mL four-necked flask equipped with a stirrer, a thermometer, a nitrogen blowing tube, and a cooling tube. After the solution was stirred and mixed uniformly, nitrogen gas was blown into the solution to remove oxygen present in the upper space of the reaction vessel (four-necked flask), and in the raw material and the reaction solvent. Subsequently, the mixture was heated to 60 to 65°C in a nitrogen atmosphere. The temperature was then maintained at 60 to 65°C for 3 hours. When the mixture was maintained at 60 to 65°C for about one hour, a mixture obtained by dissolving 1.6 g of a block copolymer of 12-hydroxystearic acid and polyoxyethylene (Hypermer B246, produced by Croda) in 2.0 g of normal hexane was added to the reaction vessel. Thereafter, the resulting slurry was heated to 100°C to distill off the normal hexane, and further dried under reduced pressure for 8 hours (115°C, 10 mmHg) to thus obtain 39 g of a white fine powder of an alkyl-modified carboxyl-group-containing water-soluble copolymer. This copolymer is sometimes referred to below as "the polymer of Production Example 4."

### Production Example 5

45 g (0.625 mol) of acrylic acid, 0.27 g of pentaerythritol tetraallyl ether, 150 g of n-hexane, and 0.081 g (0.00035 mol) of 2,2'-azobis methyl isobutyrate were placed in a 500-mL four-necked flask equipped with a stirrer, a thermometer, a nitrogen blowing tube, and a cooling tube, to prepare a reaction liquid. After the reaction liquid was stirred and mixed uniformly, nitrogen gas was blown into the solution to remove oxygen present in the upper space of the reaction vessel, and in the raw material and the solvent. Thereafter, the reaction liquid was reacted for 4 hours in a nitrogen atmosphere while the temperature was maintained at 60 to 65°C. After completion of the reaction, the resulting slurry was heated to 90°C to distill off the n-hexane, and further dried under reduced pressure for 8 hours (110°C, 10 mmHg) to thus obtain 42 g of a carboxyvinyl polymer. This carboxyvinyl polymer is sometimes referred to below as "the polymer of Production Example 5."

### Production Example 6

100 parts by mass of fully dehydrated polyethylene oxide with a number average molecular weight of 20,000, 10 parts by mass of polyoxyethylene polyoxypropylene stearyl ether (EO/PO = 50/50, Mw = 1000), and 0.2 parts by mass of dioctyltin dilaurate were placed in this ratio in a storage tank A equipped with a stirrer kept warm at 80°C. The mixture was stirred in a nitrogen gas atmosphere to obtain a uniform mixture. Separately, dicyclohexylmethane-4,4'-diisocyanate was placed in a storage tank B kept warm at 30°C, and stored in a nitrogen gas atmosphere.

Using a metering pump, the mixture in the storage tank A and dicyclohexylmethane-4,4'-diisocyanate in the storage tank B were continuously fed at a rate of 500 g/min and a rate of 11.9 g/min, respectively, to a twin-screw extruder set at 110 to 140°C (R value = 1.00). The mixture was reacted by mixing in the extruder, and the strand was discharged from the extruder outlet and was formed into pellets by using a pelletizer, thus obtaining a polyalkylene oxide-modified product. This polyalkylene oxide-modified product is sometimes referred to below as "the polymer of Production Example 6."

### Preparation of 3% Aqueous Dispersion of Polymer

291 g of ion-exchanged water was measured into a 500-ml plastic beaker equipped with a disperser (diameter: 4 cm, height: 1 cm), and 9 g of each of the polymers of Production Examples 1 to 6 was gradually added while stirring at 1,000 rpm. After the addition, the stirring speed was set at 2,500 rpm, and stirring was continued for one hour to thus obtain a 3% (mass%) aqueous dispersion of polymer.

### Preparation of Viscous Composition

The 3% aqueous dispersion of polymer, a 28.8% aqueous solution of cocoyl glutamic acid K (Amisoft CK-22, produced by Ajinomoto Healthy Supply Co., Inc.), a 30% aqueous solution of cocamidopropyl betaine (SOFTAZOLINE CPE, produced by Kawaken Fine Chemicals Co., Ltd.), and a 40% aqueous solution of lauryl glucoside (MYDOL 12, produced by Kao Corporation) were sequentially measured into a 300-ml plastic beaker equipped with four paddle blades with a diameter of 5 cm and a width of 1.5 cm. Every time each raw material was added, stirring was performed until uniform. A trisodium citrate aqueous solution prepared by dissolving trisodium citrate dihydrate (a special grade reagent, produced by Wako Pure Chemical Co., Ltd.) in ion-exchanged water was added thereto, and the mixture was stirred until uniform, thus obtaining a viscous composition.

### Example 1

A viscous composition (100 g) was prepared as described above by using 33.3 g of the 3% aqueous dispersion of the polymer of Production Example 1, 11.1 g of the 28.8% aqueous solution of cocoyl glutamic acid K, 5.6 g of the 30% aqueous solution of cocamidopropyl betaine, 10.0 g of the 40% aqueous solution of lauryl glucoside, 4.0 g of trisodium citrate dihydrate, and 36.0 g of ion-exchanged water.

### Example 2

A viscous composition (100 g) was prepared as described above by using 40.0 g of the 3% aqueous dispersion of polymer of Production Example 1, 17.4 g of the 28.8% aqueous solution of cocoyl glutamic acid K, 16.7 g of the 30% aqueous solution of cocamidopropyl betaine, 12.5 g of the 40% aqueous solution of lauryl glucoside, 4.0 g of trisodium citrate dihydrate, and 9.4 g of ion-exchanged water.

### Example 3

A viscous composition (100 g) was prepared in the same manner as in Example 2, except that the amount of the 30% aqueous solution of cocamidopropyl betaine was changed to 13.3 g, and the amount of ion-exchanged water was changed to 12.8 g.

### Example 4

A viscous composition (100 g) was prepared in the same manner as in Example 2, except that the amount of the 30% aqueous solution of cocamidopropyl betaine was changed to 10.0 g, and the amount of ion-exchanged water was changed to 16.1 g.

### Example 5

A viscous composition (100 g) was prepared in the same manner as in Example 2, except that the amount of the 30% aqueous solution of cocamidopropyl betaine was changed to 6.7 g, and the amount of ion-exchanged water was changed to 19.4 g.

### Example 7

A viscous composition (100 g) was prepared in the same manner as in Example 2, except that the amount of the 40% aqueous solution of lauryl glucoside was changed to 10.0 g, and the amount of ion-exchanged water was changed to 11.9 g.

### Example 8

A viscous composition (100 g) was prepared in the same manner as in Example 2, except that the amount of the 40% aqueous solution of lauryl glucoside was changed to 7.5 g, and the amount of ion-exchanged water was changed to 14.4 g.

### Example 9

A viscous composition (100 g) was prepared in the same manner as in Example 2, except that the amount of the 40% aqueous solution of lauryl glucoside was changed to 5.0 g, and the amount of ion-exchanged water was changed to 16.9 g.

### Example 10

A viscous composition (100 g) was prepared in the same manner as in Example 2, except that the amount of the 40% aqueous solution of lauryl glucoside was changed to 2.5 g, and the amount of ion-exchanged water was changed to 19.4 g.

### Example 11

A viscous composition (100 g) was prepared in the same manner as in Example 4, except that the 3% aqueous dispersion of polymer of Production Example 1 was replaced with the 3% aqueous dispersion of polymer of Production Example 2.

### Example 12

A viscous composition (100 g) was prepared in the same manner as in Example 2, except that the 3% aqueous dispersion of polymer of Production Example 1 was replaced with the 3% aqueous dispersion of polymer of Production Example 3.

### Example 13

A viscous composition (100 g) was prepared in the same manner as in Example 2, except that the 3% aqueous dispersion of polymer of Production Example 1 was replaced with the 3% aqueous dispersion of polymer of Production Example 4.

### Comparative Example 1

A viscous composition (100 g) was prepared in the same manner as in Example 4, except that the 3% aqueous dispersion of polymer of Production Example 1 was replaced with the 3% aqueous dispersion of polymer of Production Example 5.

### Comparative Example 2

A viscous composition (100 g) was prepared in the same manner as in Example 4, except that the 3% aqueous dispersion of polymer of Production Example 1 was replaced with the 3% aqueous dispersion of polymer of Production Example 6.

### Comparative Example 3

A viscous composition (100 g) was prepared in the same manner as in Example 2, except that the amount of the 30% aqueous solution of cocamidopropyl betaine was changed to 0.0 g, and the amount of ion-exchanged water was changed to 26.1 g.

### Comparative Example 4

A viscous composition (100 g) was prepared in the same manner as in Example 2, except that the amount of the 40% aqueous solution of lauryl glucoside was changed to 0.0 g, and the amount of ion-exchanged water was changed to 21.9 g.

### Comparative Example 5

A viscous composition (100 g) was prepared in the same manner as in Example 2, except that the amount of the 30% aqueous solution of cocamidopropyl betaine was changed to 0.0 g, the amount of the 40% aqueous solution of lauryl glucoside was changed to 0.0 g, the amount of trisodium citrate dihydrate was changed to 1.0 g, and the amount of ion-exchanged water was changed to 41.6 g.

When the obtained viscous composition was observed 15 days after the production, the upper part of the sample was solidified.

### Comparative Example 6

A composition (100 g) was prepared in the same manner as in Example 2, except that the amount of trisodium citrate dihydrate was changed to 0.0 g, and the amount of ion-exchanged water was changed to 13.4 g.

The resulting composition was not in the form of a gel; rather, it was semi-solid, with no fluidity.

### Example 14

A viscous composition (100 g) was prepared in the same manner as in Example 2, except that trisodium citrate dihydrate was replaced with (+)-sodium L-ascorbate (a special grade reagent, produced by Wako Pure Chemical Industries, Ltd.).

### Comparative Example 7

A composition (100 g) was prepared in the same manner as in Example 2, except that trisodium citrate dihydrate was replaced with sodium chloride, which is an inorganic acid (a special grade reagent, produced by Nacalai Tesque, Inc.).

The resulting composition was not in the form of a gel; rather, it was semi-solid, with no fluidity.

### Reference Example 1

A viscous composition (100 g) was prepared in the same manner as in Example 2, except that the amount of the 30% aqueous solution of cocamidopropyl betaine was changed to 0.0 g, the amount of the 40% aqueous solution of lauryl glucoside was changed to 0.0 g, the amount of trisodium citrate dihydrate was changed to 4.0 g, and the amount of ion-exchanged water was changed to 38.6 g.

Table 1 shows the formulations of the viscous compositions or compositions prepared in the Examples, Comparative Examples, and Reference Example. In Table 1, the amount of each component is based on percent by mass. Table 1 also shows the measurement results of the viscosity (mPa·s), the transmittance (%), and the pH of each composition.

**Table 1**

| | Type of polymer | Polymer | Cocoyl glutamic acid K | Cocamidopropyl betaine | Lauryl glucoside | Sodium citrate | Sodium ascorbate | Sodium chloride | Viscosity | Transmittance | pH |
|---|---|---|---|---|---|---|---|---|---|---|---|
| Ex. 1 | Prod. Ex. 1 | 1.0 | 3.2 | 1.68 | 4.0 | 4.0 | - | - | 2,620 | 65.8 | 5.5 |
| Ex. 2 | Prod. Ex. 1 | 1.2 | 5.0 | 5.0 | 5.0 | 4.0 | - | - | 8,250 | 35.8 | 5.6 |
| Ex. 3 | Prod. Ex. 1 | 1.2 | 5.0 | 4.0 | 5.0 | 4.0 | - | - | 6,600 | 35.8 | 5.7 |
| Ex. 4 | Prod. Ex. 1 | 1.2 | 5.0 | 3.0 | 5.0 | 4.0 | - | - | 3,440 | 44.1 | 5.6 |
| Ex. 5 | Prod. Ex. 1 | 1.2 | 5.0 | 2.0 | 5.0 | 4.0 | - | - | 1,540 | 61.8 | 5.6 |
| Ex. 7 | Prod. Ex. 1 | 1.2 | 5.0 | 5.0 | 4.0 | 4.0 | - | - | 4,340 | 38.2 | 5.6 |
| Ex. 8 | Prod. Ex. 1 | 1.2 | 5.0 | 5.0 | 3.0 | 4.0 | - | - | 2,890 | 48 | 5.7 |
| Ex. 9 | Prod. Ex. 1 | 1.2 | 5.0 | 5.0 | 2.0 | 4.0 | - | - | 1,850 | 59 | 5.7 |
| Ex. 10 | Prod. Ex. 1 | 1.2 | 5.0 | 5.0 | 1.0 | 4.0 | - | - | 1,200 | 68.3 | 5.7 |
| Ex. 11 | Prod. Ex. 2 | 1.2 | 5.0 | 3.0 | 5.0 | 4.0 | - | - | 2,050 | Cloudy (1.2) | 5.6 |
| Ex. 12 | Prod. Ex. 3 | 1.2 | 5.0 | 5.0 | 5.0 | 4.0 | - | - | 5,800 | Cloudy (1.1) | 5.5 |
| Ex. 13 | Prod. Ex. 4 | 1.2 | 5.0 | 5.0 | 5.0 | 4.0 | - | - | 1,920 | Cloudy (0.3) | 5.5 |
| Com. Ex. 1 | Prod. Ex. 5 | 1.2 | 5.0 | 3.0 | 5.0 | 4.0 | - | - | 350 | Cloudy (0.3) | 5.8 |
| Com. Ex. 2 | Prod. Ex. 6 | 1.2 | 5.0 | 3.0 | 5.0 | 4.0 | - | - | 100 or less | With deposit (94,0) | 6.5 |
| Com. Ex. 3 | Prod. Ex. 1 | 1.2 | 5.0 | - | 5.0 | 4.0 | - | - | 530 | 76.1 | 5.6 |
| Com. Ex. 4 | Prod. Ex. 1 | 1.2 | 5.0 | 5.0 | - | 4.0 | - | - | 480 | 73.4 | 5.8 |
| Com. Ex. 5 | Prod. Ex. 1 | 1.2 | 5.0 | - | - | 1.0 | - | - | Upper part solidified (15 days after production) | - | 5.8 |
| Com. Ex. 6 | Prod. Ex. 1 | 1.2 | 5.0 | 5.0 | 5.0 | - | - | - | Solidified | Cloudy (0.2) | 5.2 |
| Ex. 14 | Prod. Ex. 1 | 1.2 | 5.0 | 5.0 | 5.0 | - | 4.0 | - | 11,750 | 31.3 | 5.2 |
| Com. Ex. 7 | Prod. Ex 1 | 1.2 | 5.0 | 5.0 | 5.0 | - | - | 4.0 | Solidified | Cloudy (0.1) | 4.8 |
| Ref. Ex. 1 | Prod. Ex. 1 | 1.2 | 5.0 | - | - | 4.0 | - | - | 2,660 | 93.2 | 5.7 |

## Claims

1. A viscous composition comprising
(A) a copolymer obtained by polymerizing at least 100 parts by mass of a (meth)acrylic acid and 0.5 to 5 parts by mass of a (meth)acrylic acid alkyl ester having an alkyl group with 10 to 30 carbon atoms,
(B) an amino acid-based surfactant,
(C) a betaine,
(D) an alkyl glycoside, and
(E) an organic acid salt,
wherein the mass content of (B) is equal to or greater than the mass content of (C).

2. The viscous composition according to claim 1, having a viscosity of 1000 mPa·s or more.

3. The viscous composition according to claim 1 or 2, wherein (A) is a copolymer obtained by polymerizing at least 100 parts by mass of a (meth)acrylic acid and 0.5 to 5 parts by mass of a (meth)acrylic acid alkyl ester having an alkyl group with 18 to 24 carbon atoms.

4. The viscous composition according to any one of claims 1 to 3, wherein (A) is a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and a compound with two or more ethylenically unsaturated groups.

5. The viscous composition according to claim 4, wherein (A) is a copolymer obtained by polymerizing 100 parts by mass of a (meth)acrylic acid, 0.5 to 5 parts by mass of a (meth)acrylic acid alkyl ester having an alkyl group with 18 to 24 carbon atoms, and 0.001 to 1 part by mass of a compound with two or more ethylenically unsaturated groups.

6. The viscous composition according to claim 4 or 5, wherein the compound with two or more ethylenically unsaturated groups is at least one member selected from the group consisting of pentaerythritol allyl ether, tetraallyloxyethane, triallyl phosphate, and polyallylsaccharose.

7. The viscous composition according to any one of claims 1 to 6, wherein the content mass ratio of (B) and (C) is 1 to 5:1.

8. The viscous composition according to any one of claims 1 to 7, wherein (B) is a compound represented by the formula: 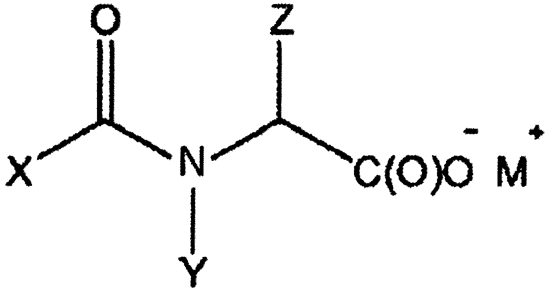
wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O-M⁺, -CH₂COOH, or -(CH₂)₂COOH, and
M represents sodium, potassium, ammonium, or triethanolammonium.

9. The viscous composition according to any one of claims 1 to 7, wherein (C) is at least one member selected from the group consisting of compounds represented by the formula: 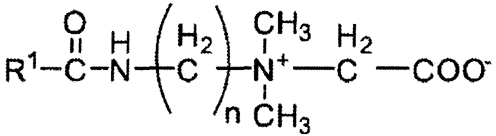 wherein R¹ represents a linear or branched alkyl group with 5 to 22 carbon atoms, and n represents 1 to 6, and
compounds represented by the formula: 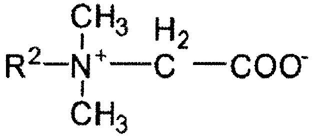 wherein R² represents a linear or branched alkyl group with 1 to 22 carbon atoms.

10. The viscous composition according to any one of claims 1 to 7,
wherein (B) is a compound represented by the formula: 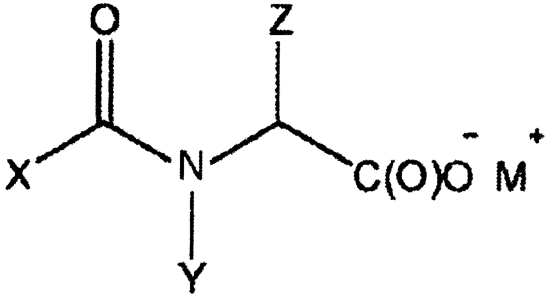
wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC (NH)NH₂, -CH₂C(O)O⁻M⁺, - (CH₂)₂C(O)O⁻M⁺, -CH₂COOH, or - (CH₂)₂COOH, and
M represents sodium, potassium, ammonium, or triethanolammonium; and
wherein (C) is at least one member selected from the group consisting of compounds represented by the formula: 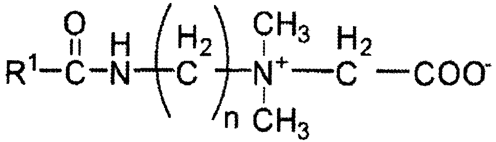 wherein R¹ represents a linear or branched alkyl group with 5 to 22 carbon atoms, and n represents 1 to 6, and compounds represented by the formula: 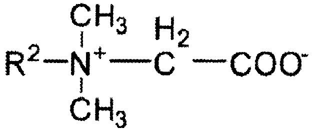 wherein R² represents a linear or branched alkyl group with 1 to 22 carbon atoms.

11. The viscous composition according to any of claims 1 to 10, wherein (D) is an alkyl glucoside in which one linear alkyl group with 5 to 22 carbon atoms and one glucose are condensed.

12. The viscous composition according to any of claims 1 to 11, wherein (E) is at least one member selected from the group consisting of carboxylic acid metal salts and ascorbic acid metal salts.

13. The viscous composition according to claim 6,
wherein the content mass ratio of (B) and (C) is 1 to 5:1, and the compound with two or more ethylenically unsaturated groups is at least one member selected from the group consisting of pentaerythritol allyl ether, tetraallyloxyethane, triallyl phosphate, and polyallylsaccharose,
wherein
(B) is a compound represented by the formula: 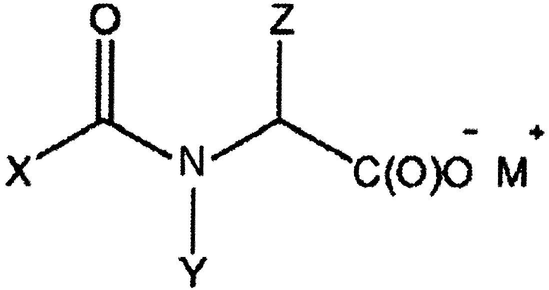
wherein X represents a saturated or unsaturated hydrocarbon group with 5 to 22 carbon atoms,
Y represents a hydrogen atom or a methyl group,
Z represents a hydrogen atom, -CH₃, -CH(CH₃)₂, -CH₂CH(CH₃)₂, -CH(CH₃)CH₂CH₃, -CH₂C₆H₅, -CH₂C₆H₄OH, -CH₂OH, -CH(OH)CH₃, -(CH₂)₄NH₂, - (CH₂)₃NHC(NH)NH₂, -CH₂C(O)O⁻M⁺, -(CH₂)₂C(O)O⁻M⁺, -CH₂COOH, or - (CH₂) ₂COOH, and
M represents sodium, potassium, ammonium, or triethanolammonium, wherein
(C) is at least one member selected from the group consisting of compounds represented by the formula: 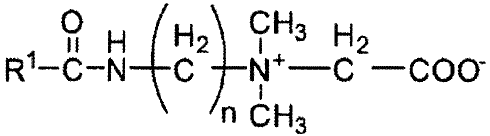
wherein R¹ represents a linear or branched alkyl group with 5 to 22 carbon atoms, and n represents 1 to 6, and
compounds represented by the formula: 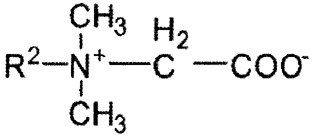
wherein R² represents a linear or branched alkyl group with 1 to 22 carbon atoms,
wherein
(D) is an alkyl glucoside in which one linear alkyl group with 5 to 22 carbon atoms and one glucose are condensed, and
wherein
(E) is at least one member selected from the group consisting of carboxylic acid metal salts and ascorbic acid metal salts.
